# DEMANDE DE BREVET EUROPEEN

(11) **EP 0 684 474 A1**
(43) Date de publication de la demande: **29.11.1995**
(21) Numéro de dépôt: 95401219.1
(22) Date de dépôt: 24.05.1995
(51) Int. Cl.: G01N 33/546, C12Q 1/68, G01N 33/58, G01N 33/53

(54) **Procédé et dispositif pour la mise en évidence d'un analyte dans un échantillon**

(30) Priorité: 25.05.1994 FR 9406326
(71) Demandeur: SOCIETE DE RECHERCHE ET DE DEVELOPPEMENT EN ACTIVATION ET COMMUNICATION CELLULAIRE, 06610 La Gaude (FR)
(72) Inventeur: Garcia Gonzales, Mercedes, F-94380 Bonneuil sur Marne (FR); Sieprawski, Elvire, F-38170 Seyssinet (FR); Bayer, Victor, F-06100 Nice (FR)
(74) Mandataire: Warcoin, Jacques

(57) **Abrégé**

L'invention concerne un procédé de mise en évidence d'un analyte dans un échantillon et un dispositif permettant de mettre en oeuvre le procédé.

Le dispositif (1) est remarquable en ce qu'il comporte un volume (4) tubulaire, présentant une zone inférieure, et une zone supérieure (5) de lecture, limité à sa partie inférieure par une membrane (2) perméable aux liquides, dans lequel coulisse un piston (3), ledit volume contenant dans la zone inférieure une phase solide (6) constituée des particules de densité supérieure et inférieure à l'échantillon.

## Description

La présente invention concerne un procédé de mise en évidence d'un analyte en solution dans un liquide du type comportant la fixation d'éléments de reconnaissance sur les éléments complémentaires de l'analyte.

Elle concerne également un dispositif permettant de mettre en oeuvre un tel procédé.

Par l'expression "éléments de reconnaissance" on entend tout moyen permettant, par liaison covalente ou non covalente réversible ou non, la formation d'un complexe avec un ou plusieurs éléments complémentaires.

Ces éléments de reconnaissance seront donc divers tels qu'antigènes, anticorps, monobrins d'acides nucléiques, sites spécifiques de récepteur ou de ligand.

Les éléments complémentaires peuvent être l'analyte lui-même et sont choisis parmi les antigènes, les anticorps, les monobrins d'acides nucléiques, les sites spécifiques de récepteur ou de ligand correspondants.

Un des objets de la présente invention est de proposer un nouveau procédé ainsi qu'un dispositif pour le mettre en oeuvre permettant la réalisation, avec une prise d'essai réduite, d'un test de détection de substances, espèces chimiques notamment substances biologiques, en particulier à motifs antigéniques ou pouvant s'hybrider avec des substances complémentaires comme les acides nucléiques.

Selon un mode général de réalisation, l'invention est caractérisée en ce que :
- un échantillon de ladite solution est mis en contact, dans une chambre de réaction, avec une phase solide fluide qui comporte des particules de densité inférieure (Pfd) à celle du milieu liquide et des particules de densité supérieure (PFd) à celle du milieu liquide, sur lesquelles ont été fixés soit des éléments de reconnaissance, soit l'analyte ou des éléments complémentaires des éléments de reconnaissance propres à l'analyte, au moins l'une des deux catégories de particules étant associées auxdits éléments de reconnaissance, en ce que les particules de chaque catégorie présentent un aspect visuel différent,
et en ce qu'après réaction, le résultat est contrôlé au voisinage d'une extrémité de la chambre de réaction. Par "extrémité", on entend la surface ou le fond, de préférence la surface.

Selon une variante, l'invention est caractérisée en ce que :
- un échantillon de ladite solution est mis en contact, dans un volume limité tenant lieu de chambre de réaction, avec une phase solide fluide qui comporte des particules de densité inférieure (Pfd) à celle du milieu liquide et des particules de densité supérieure (PFd) à celle du milieu liquide, sur lesquelles ont été fixés soit des éléments de reconnaissance, soit l'analyte ou des éléments complémentaires des éléments de reconnaissance propres à l'analyte, au moins l'une des deux catégories de particules étant associées auxdits éléments de reconnaissance, en ce que les particules de chaque catégorie présentent un aspect visuel différent,
   et en ce que
- après réaction on ajoute au volume tenant lieu de chambre de réaction une quantité déterminée de liquide de façon à permettre la lecture du résultat au voisinage de la surface

Par "volume limité" on entend un volume qui est suffisamment proche du volume occupé par les particules pour que celles-ci ne puissent, sous la simple action de leurs densités respectives, se déplacer et se diriger, pour les unes, vers la surface et, pour les autres, vers le fond lorsque le milieu liquide est introduit.

Ce volume constitue la chambre de réaction permettant l'échange jusqu'à l'équilibre entre les différents réactifs.

L'ajout supplémentaire de liquide, qui est le plus souvent le liquide dans lequel a été solubilisé, dissous ou extrait l'analyte de l'échantillon, est suffisant pour permettre la remontée éventuelle de la phase solide fluide à la surface. Cet ajout permet également de dégager une zone de lecture au voisinage de la surface.

L'aspect visuel différent peut être obtenu soit par une couleur différente pour chaque catégorie de particules, soit par le caractère non coloré (ou blanc) d'une catégorie et le caractère coloré de l'autre catégorie.

Lorsque les complexes entre les particules des différentes catégories ont pu se former, les particules de faible diamètre recouvrent les particules de diamètre plus important et l'aspect constaté est sensiblement celui des particules de faible diamètre. Par exemple, lorsque les particules de forte densité sont d'un diamètre beaucoup plus faible que celui des particules de faible densité, elles recouvrent les particules de faible densité et l'aspect constaté est sensiblement celui des particules de forte densité.

Bien que la présente description sera explicitée plus en détail dans le cas où les particules de densité supérieure présentent le diamètre le plus faible, l'invention s'applique mutatis mutandis aux particules de densité supérieure qui présentent le diamètre le plus important.

Lorsque l'on utilise l'expression "aspect des particules", celle-ci désigne la plupart du temps l'aspect de la couche formée par un agrégat de particules.

Selon les cas, la densité des complexes formés par les particules des différentes catégories et éventuellement l'analyte, appelés ci-après "complexes", est soit supérieure, soit inférieure à celle de la solution. Cette densité sera fonction de la densité de chaque catégorie de particules, mais également du nombre de particules de faible diamètre capables d'être associées à une particule de diamètre plus important.

De façon générale, les particules de forte densité occupent 60 à 95 % du volume de la phase solide et les particules de faible densité entre 5 et 40 % dans le cas où les particules de forte densité présentent le diamètre le plus faible.

Dans le cas de la présence de plusieurs analytes dans la solution, les particules seront revêtues des éléments de reconnaissance et/ou éléments complémentaires correspondants. Cette répartition pourra être homogène (chaque particule n'étant revêtue que d'une sorte d'éléments de reconnaissance et/ou éléments complémentaires) ou hétérogène (les particules étant revêtues d'un mélange des différents éléments de reconnaissance et/ou éléments complémentaires).

Selon une première variante du procédé, celui-ci est caractérisé en ce que les éléments de reconnaissance sont fixés sur les particules de densité inférieure (Pfd) à celle du milieu liquide et sur les particules de densité supérieure (PFd) à celle du milieu liquide.

Ce procédé fait donc appel à la méthode appelée communément "sandwich" et est particulièrement approprié lorsque plusieurs éléments complémentaires sont présents sur l'analyte.

Ces éléments complémentaires peuvent être identiques ou différents et peuvent être constitués, par exemple, par plusieurs déterminants antigéniques identiques ou différents.

Lorsque les déterminants antigéniques sont différents, les particules présentent les anticorps correspondants à leur surface.

Dans tous les cas, les anticorps pourront être mono ou polyclonaux.

Dans le cas où les complexes présentent une densité inférieure à celle de la solution et où les particules de forte densité présentent le diamètre le plus faible, lorsque l'analyte est présent, apparaît à la surface une couche de couleur correspondant substantiellement à celle des particules de densité supérieure (la couleur pouvant être atténuée selon la quantité de particules de forte densité présentes à la surface des particules de faible densité qui est fonction de la concentration en analyte).

Dans le cas où les complexes présentent une densité inférieure à celle de la solution et lorsque l'analyte est absent de la solution, apparaît à la surface une couche de couleur correspondant à celle des particules de densité inférieure (Pfd) qui sont par conséquent non complexées.

Cette méthode de la première variante est préférée.

Dans le cas où les complexes présentent une densité supérieure à celle de la solution, lorsque l'analyte est absent de la solution, apparaît à la surface une couche de couleur correspondant à celle des particules de densité inférieure.

Dans le cas où les complexes présentent une densité supérieure à celle de la solution et lorsque l'analyte est présent dans la solution, aucune couche n'apparaît à la surface.

Selon une seconde variante du procédé selon l'invention, celui-ci est caractérisé en ce que l'analyte, ou les éléments complémentaires de celui-ci, est fixé sur les particules de densité supérieure (PFd) à celle du milieu liquide ou les particules de densité inférieure (Pfd) à celle du milieu liquide.

Dans le cas où les complexes présentent une densité inférieure à celle de la solution, lorsque l'analyte est présent, apparaît à la surface une couche de couleur correspondant à celle des particules de densité inférieure (Pfd) qui sont par conséquent non complexées. Cette couleur peut être mélangée à la couleur des particules de forte densité au cas où la quantité d'analyte est faible.

Dans le cas où les complexes présentent une densité inférieure à celle de la solution, les particules de forte densité présentant le diamètre le plus faible, et lorsque l'analyte est absent, apparaît à la surface une couche de couleur correspondant substantiellement à celle des particules de densité supérieure (la couleur pouvant être atténuée selon la quantité de particules de forte densité présentes à la surface des particules de faible densité).

Cette méthode selon la seconde variante est préférée.

Dans le cas où les complexes présentent une densité supérieure à celle de la solution, lorsque l'analyte est présent, une couche de couleur correspondant à celle des particules de faible densité apparaît à la surface. La couleur peut néanmoins être atténuée lorsque la concentration en échantillon est faible.

Dans le cas où les complexes présentent une densité supérieure à celle de la solution, lorsque l'analyte est absent, aucune couche n'apparaît à la surface.

Ce procédé fait donc appel à la réaction de compétition (ou de déplacement) et est particulièrement approprié lorsqu'un élément complémentaire est présent sur l'analyte. Cet élément complémentaire peut être un déterminant antigénique et l'élément de reconnaissance des anticorps mono ou polyclonaux.

De manière générale, les éléments de reconnaissance sont avantageusement des anticorps, notamment de type IgM, IgG, IgA, IgE et de préférence de type IgM.

Pratiquement, l'analyte est solubilisé dans une phase solvant aqueuse, organique ou hydro-organique selon la nature de l'analyte, par exemple tampons phosphate de sodium et potassium, bicarbonate de sodium, acétate de sodium, Tris-NaCl, MES ou mélanges alcool-tampon aqueux, mélanges diméthylsulfoxyde-tampon aqueux, diméthylformamide-tampon, dioxane-tampon aqueux ou solutions contenant des détergents (cationiques, anioniques ou zwitterions) destinés à mieux solubiliser l'analyte à partir de l'échantillon. De préférence les tampons phosphate de sodium et potassium sont utilisés.

Après mélange éventuellement sous agitation, le prélèvement est effectué de façon à ce que les particules soient lentement mises en contact avec la solution. Le temps de réaction peut être variable selon les substances en jeu, mais n'excèdera pas le plus souvent quelques minutes.

Sur un plan pratique, afin de faciliter la lecture, les particules de faible et/ou de forte densité selon les cas sont traitées pour être identifiées.

Le traitement des particules pour permettre leur identification consiste généralement à les teindre de manière connue ou à les rendre fluorescentes. En général, une seule des deux catégories de particules est teintée, l'autre restant non teintée.

Selon un autre mode de réalisation, les particules de densité inférieure présentent un diamètre inférieur à 1 µm et les particules de densité supérieure comprise entre 10 et 50 µm.

Selon un mode préféré de réalisation, les particules de densité inférieure présentent un diamètre de 30 à 120 µm, de préférence 40 à 100 µm, tandis que les particules de densité supérieure présentent un diamètre inférieur à 10 µm, avantageusement inférieur à 5 µm, par exemple 1 à 2 µm.

Les microbilles de densité supérieure sont choisies parmi les :
- particules fluorescentes
- particules magnétiques coloriées noir Fe₃O₄, rouge Fe₂O₃,
- pigments de cadmium rouge,
- pigments de cadmium jaune,
- pigments de phtalocyanine
- particules de latex-polystyrène bleues, rouges,
- pigments cosmétiques (à base de mica),
- pigments pour l'industrie automobile,
- or colloïdal
- Fractogel® TSK colorant triazine (bleu, rouge, vert, orange) de diamètre 32-63 µm.

Les microbilles de densité inférieure sont choisies parmi le :
- polyéthylène radicalaire,
- polyacrylamide,
- polystyrène expansé,
- les matériaux de remplissage de colonne chromatographie gaz-liquide, comme les résines de polystyrène et de divinylbenzène, réticulées de diamètre 100-200 µm,
- billes de verre creuses.

Les microbilles à base de silice doivent être revêtues par un agent de silanisation avant greffage des éléments de reconnaissance ou des analytes. Ces agents de silanisation sont soit des aminosilanes, soit des thiolsilanes, soit des epoxysilanes ou autres.

Parmi ceux-ci, on peut mentionner ceux de la liste suivante :
- triethoxysilyl-3-propylamine,
- triméthoxysilyl-3-propylamine,
- 4-aminobutyl triethoxysilane,
- 4-aminobutyl diméthoxysilane,
- mercaptométhyl-diméthylethoxysilane,
- triméthoxysilyl-3-propanethiol-1
- 3-glycidoxypropyl triméthoxysilane,
- N-(triméthylsilyl)imidazole.

Les autres particules sont revêtues d'un polymère synthétique du type polypeptide comme le poly(glu:lys) ou similaire ou oligonucléotide ou un haptène ou de polymères naturels comme la protamine, la sérum albumine bovine, etc.

Les agents de couplage covalents sont avantageusement choisis parmi :
- les carbodiimides,
- les esters d'hydroxysuccinimidyle
- le glutaraldéhyde,
- le maleimide hydroxysuccimide,
- l'anhydride succinique
- le périodate

pour les polypeptides et les haptènes, et sont choisis parmi :
- le periodate
- réactifs photoactivables
- réactifs photopolymérisables ou électropolymérisables.

pour les oligonucléotides.

De préférence, les particules de faible densité sont constituées de billes de verre creuses.

De préférence, les particules de densité supérieure sont constituées de pigments cosmétiques à base de mica.

Les microbilles de densité supérieure ont une densité supérieure à 200 %, généralement comprise entre 200 et 600 % de la densité de la phase solvant.

Les microbilles de densité inférieure ont une densité généralement comprise entre 10 et 30 % de la densité de la phase solvant.

La masse surfacique des éléments de reconnaissance ou des analytes qui recouvrent les microbilles est généralement comprise entre 0,1 et 5 µg/cm².

L'invention a également pour objet un dispositif pour mettre en oeuvre le procédé décrit précédemment, caractérisé en ce qu'il comporte un volume tubulaire, présentant une zone inférieure, une zone médiane et une zone supérieure de lecture, limité à sa partie inférieure par une membrane perméable aux liquides, dans lequel coulisse un piston, ledit volume contenant dans la zone inférieure la phase solide constituée des particules de densité inférieure et supérieure.

De préférence, la membrane est constituée d'un matériau choisi dans le groupe constitué par l'acétate de cellulose, le polyvinyldifluorobenzène, le nitrate de cellulose, la fibre de verre. Le choix de la membrane est fonction du solvant.

Selon une variante avantageuse, le dispositif comporte un fourreau dans lequel, dans sa partie supérieure, s'étendent un ou plusieurs tubes, chacun contenant une quantité appropriée de particules et dans lequel dans sa partie inférieure, coulisse un réservoir contenant la solution à analyser de façon à ce que par translation, le réservoir permette à la solution de remplir les tubes et en ce que après contact entre les particules et la solution le résultat puisse être contrôlé dans la zone de lecture.

De préférence, le réservoir est limité par un film qui se déchire lorsqu'il rentre en contact avec les tubes sous l'effet du déplacement du piston.

D'autres caractéristiques et avantages de ce procédé ainsi que du dispositif pour mettre en oeuvre le procédé ressortiront encore de la description qui suit. Cette description est purement illustrative et non limitative. Elle doit être lue en regard des dessins annexés sur lesquels :
- La figure 1 est une vue schématique en coupe longitudinale d'un dispositif conforme à l'invention.
- Les figures 2 à 6 illustrent une mise en oeuvre du procédé selon l'invention et représentent des vues schématiques en perspective du dispositif ou d'un élément de ce dispositif conforme à l'invention.
- La figure 7 est une vue en coupe longitudinale de deux demi-coupes d'un second dispositif selon l'invention.

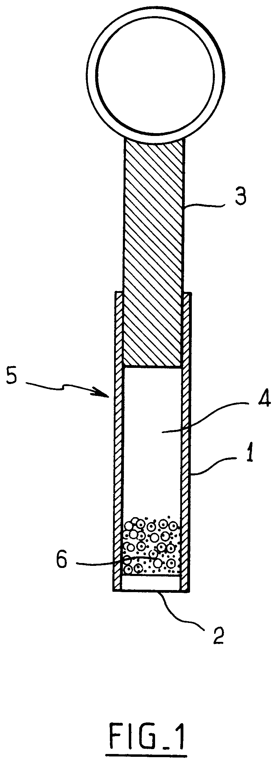

Selon la figure 1, le dispositif conforme à l'invention est formé d'une structure tubulaire 1 terminée à son extrémité inférieure par une membrane-filtre 2 en acétate de cellulose. Par l'extrémité supérieure de la structure tubulaire 1, coulisse un piston 3 à l'intérieur du volume 4 limité par ladite structure tubulaire. Le piston 3 est pourvu à son extrémité supérieure d'un moyen de préhension qui peut être un anneau solidaire dudit piston. Le volume limité par la structure tubulaire 1 comprend une zone inférieure dans laquelle sont présents le mélange des particules de forte et faible densité et qui constitue la chambre de réaction, ainsi qu'une zone supérieure dite de lecture 5.

Selon un exemple, les billes non teintées de densité inférieure à celle de la phase solvant ont un diamètre compris entre 40 et 100 µm et sont revêtues d'un anticorps de souris de classe IgM, selon une masse surfacique de 2,7 µg/cm². Ces microbilles sont creuses, en silice et ont une densité de 0,15.

Les microbilles teintées sont revêtues d'un anticorps de souris de classe IgM selon une masse surfacique de 1 µg/cm² et sont en mica . Elles présentent une densité de 2,5.

La phase solide 6 comprend en volume entre 60 et 95% de microbilles teintées et de 5 à 40% de microbilles non teintées, de préférence respectivement 80 et 20 %.

Les complexes obtenus auront dans ce cas une densité inférieure à celle de la solution.

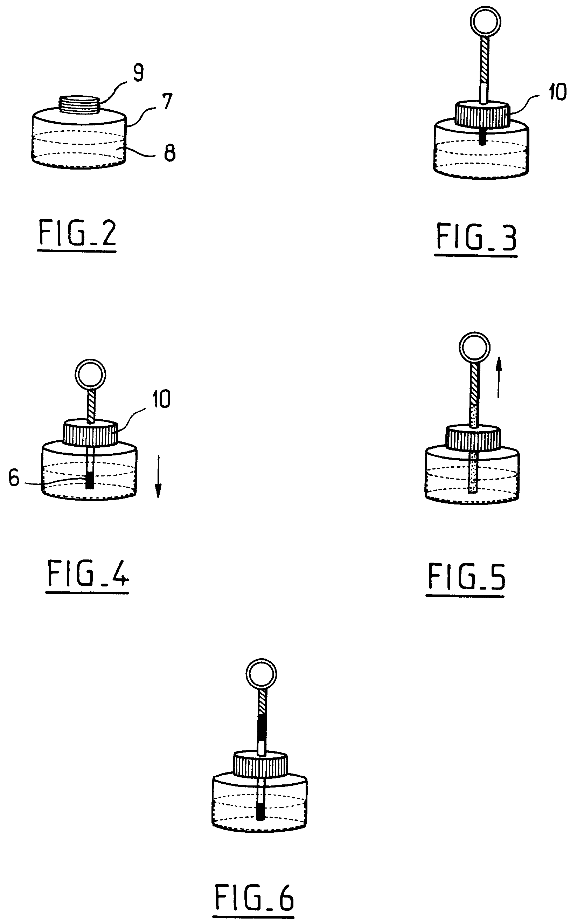

Selon la figure 2, on introduit la substance à analyser dans un flacon 7 contenant un solvant 8, ledit flacon étant pourvu d'un col fileté 9.

Selon la figure 3, le dispositif décrit à la figure 1 est solidaire du bouchon 10 destiné à fermer le flacon 7 au moyen d'un orifice (non figuré) situé au centre du bouchon et au travers duquel la structure tubulaire peut glisser tout en étant maintenue par l'orifice lorsqu'aucune force lui est appliquée.

Selon la figure 4, le piston étant maintenu en position abaissée, on immerge la zone inférieure de la structure tubulaire contenant la phase solide 6 en faisant glisser celle-ci dans l'orifice central du bouchon.

Selon la figure 5, le piston est lentement remonté pour remplir la zone inférieure ou chambre de réaction par aspiration du volume de solution correspondant à la partie inférieure de course du piston.

Au bout de quelques minutes, selon la figure 6, le piston est remonté jusqu'à l'extrémité supérieure de façon à ce que la solution remplisse le volume ainsi dégagé et il apparaît un surnageant teinté constitué de l'ensemble de particules de faible densité et de forte densité. Dans le cas où l'analyte est absent, apparaît uniquement une couche de particules de faible densité au niveau de la zone de lecture. Selon une gamme étalon établie auparavant avec plusieurs échantillons connus, on peut déterminer aisément la présence ou non d'analyte ainsi que visuellement la quantité approximative de cette substance.

Un deuxième exemple a été réalisé avec un analyte ne comportant qu'un seul épitope tel que la biotine, selon la méthode de compétition.
- Des anticorps polyclonaux anti-biotine ont été fixés de façon covalente sur des microbilles de verre creuses (particules de faible densité, blanches) péalablement traitées avec de l'aminoethoxysilane,
- Un dérivé de la biotine a été fixé de façon covalente sur des pigments bleus de mica (particules de forte densité) préalablement traités avec le polymère synthétique poly(glu:lys),
- Les deux types de microbilles précédemment décrites ont été mis en contact dans la chambre de réaction. Une couleur bleue apparaît dans la zone de lecture.
- Dans une seconde expérience dans laquelle de la biotine libre est ajoutée dans le milieu liquide, la couleur bleue n'est plus visible dans la zone de lecture.

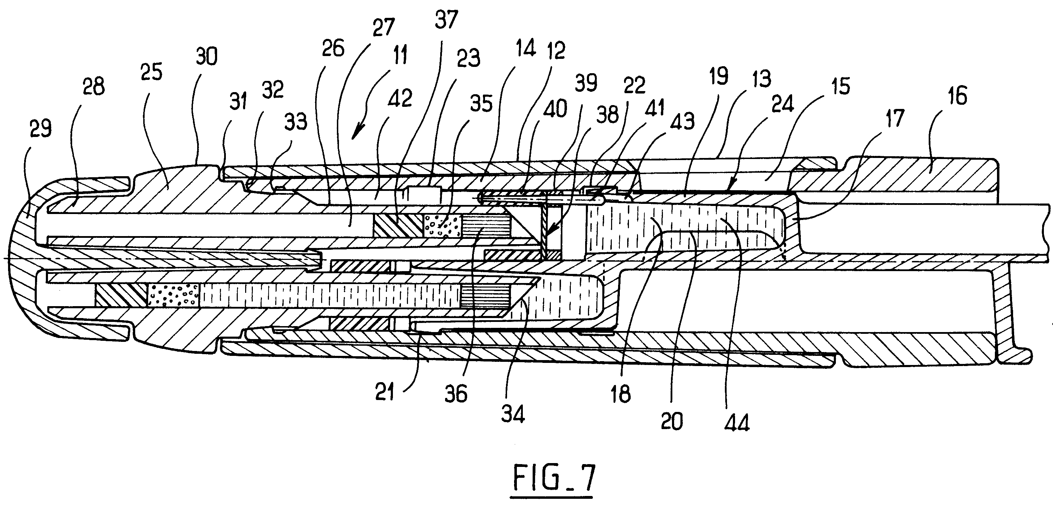

Selon la figure 7, le dispositif 11 comprend un fourreau 12 muni d'une lumière oblongue 13 dont les bords sont biseautés à proximité de sa base, dans lequel est logé un boîtier 14, présentant une lumière oblongue 15 correspondante à celle du fourreau, muni à sa base d'une surépaisseur 16 s'appuyant sur le bord du fourreau. A l'intérieur, coulisse à partir de la base un piston 17 terminé par un réservoir 18 non fermé et dont la paroi cylindrique 19 comporte une lumière oblongue 20 correspondante à celle du fourreau et du boîtier. Le bord de la paroi comporte également des pattes d'encliquetage 21 permettant de maintenir le piston au repos par des encoches 22 situées à la base du boîtier 14 et permettant d'arrêter la course du piston environ à mi-parcours par encliquetage dans des encoches 23 prévues à cet effet dans le boîtier. La paroi du réservoir est pourvue d'un joint 24 assurant l'étanchéité vis-à-vis du boîtier lorsque la lumière 20 n'est plus en regard avec la lumière 15.

Un corps 25 occupant la partie supérieure du boîtier est pourvu de trois tubes 26 s'étendant à l'intérieur du boîtier délimitant trois conduits longitudinaux 27 traversant ledit corps, et disposés à égale distance angulaire, et comporte une tête 28 sur laquelle un capuchon 29 vient s'adapter, prolongée après un épaulement par une zone élargie 30 de lecture suivie d'un premier décrochement 31 sur lequel prend appui le bord supérieur du fourreau puis d'un autre décrochement 32 délimitant avec un rebord droit 33 en regard une encoche dans laquelle vient se bloquer un décrochement droit interne du boîtier ce qui assure la fixation du corps interne au boîtier.

Les conduits sont terminés à leur base en biseau 34 et comportent une quantité de microbilles 35 confinées du côté de l'entrée par un filtre 36 et à l'opposé par un joint 37.

Entre le piston et le corps interne est présent un film 38 fixé sur une bague 39 coulissante, située du côté du piston, le film étant solidaire de joints 40 coulissant dans l'espace 42 délimité par les tubes et la paroi interne du boîtier. Un téton 41 normal à la membrane épouse la forme d'une gouttière 43 ménagée dans la paroi interne de façon à ce que par coulissement du piston, le fond de la gouttière appuie sur le sommet du téton qui transmet le mouvement au film qui à son tour se déplace.

La lumière 20 du réservoir étant placée en correspondance avec la lumière du boîtier, le réservoir est rempli d'une quantité de solution 44 à tester puis par un mouvement de rotation le réservoir est verouillé en position fermée.

Le dispositif tenu verticalement, le piston est déplacé, le fond de la gouttière appuie sur le téton 41 qui pousse le film. Le film est déchiré par la pointe des biseaux 34 et le liquide à tester peut pénétrer dans les conduits au travers des filtres puis des microbilles 35. Le réservoir est bloqué en fin de course par l'encliquetage des pattes 21 dans les encoches 23.

Par agitation, le contact intime des microbilles avec la solution est assurée et le résultat est contrôlé par lecture au niveau de la zone 30.

Il est également possible de quantifier la mesure visuelle par un appareillage approprié.

De même, le procédé peut être automatisé.

L'invention n'est pas limitée au procédé décrit dans la description ci-dessus mais s'étend aux équivalents permettant d'atteindre le même résultat.

## Revendications

**1)** Procédé de mise en évidence de la présence d'un analyte en solution dans un liquide du type comportant la fixation d'éléments de reconnaissance sur les éléments complémentaires de l'analyte, caractérisé en ce que :
- un échantillon de ladite solution est mis en contact, dans une chambre de réaction, avec une phase solide fluide qui comporte des particules de densité inférieure (Pfd) à celle du milieu liquide et des particules de densité supérieure (PFd) à celle du milieu liquide, sur lesquelles ont été fixés soit des éléments de reconnaissance, soit l'analyte ou des éléments complémentaires des éléments de reconnaissance propres à l'analyte au moins l'une des deux catégories de particules étant associées auxdits éléments de reconnaissance, en ce que les particules de chaque catégorie présentent un aspect visuel différent et en ce que,
- après réaction le résultat est contrôlé au voisinage d'une extrémité de la chambre de réaction.

**2)** Procédé de mise en évidence de la présence d'un analyte en solution dans un liquide selon la revendication 1, caractérisé en ce que :
- un échantillon de ladite solution est mis en contact, dans un volume limité tenant lieu de chambre de réaction, avec une phase solide fluide qui comporte des particules de densité inférieure (Pfd) à celle du milieu liquide et des particules de densité supérieure (PFd) à celle du milieu liquide, sur lesquelles ont été fixés soit des éléments de reconnaissance, soit l'analyte ou des éléments complémentaires des éléments de reconnaissance propres à l'analyte au moins l'une des deux catégories de particules étant associées auxdits éléments de reconnaissance, en ce que les particules de chaque catégorie présentent un aspect visuel différent et en ce que,
- après réaction on ajoute au volume tenant lieu de chambre de réaction une quantité déterminée de liquide de façon à permettre la lecture du résultat au voisinage de la surface.

**3)** Procédé selon la revendication 1, caractérisé en ce que les éléments de reconnaissance sont fixés sur les particules de densité supérieure (PFd) à celle du milieu liquide et sur les particules densité inférieure (Pfd) à celle du milieu liquide.

**4)** Procédé selon la revendication 3, caractérisé en ce que le complexe formé par l'association des particules de densité inférieure et supérieure présente une densité inférieure à celle de la solution et en ce que l'analyte présente plusieurs éléments complémentaires.

**5)** Procédé selon la revendication 1, caractérisé en ce que l'analyte ou les éléments complémentaires de celui-ci, est fixé sur les particules de densité supérieure (PFd) à celle du milieu liquide ou sur les particules de densité inférieure(Pfd) à celle du milieu liquide.

**6)** Procédé selon la revendication 5, caractérisé en ce que le complexe formé par l'association des particules de densité inférieure et supérieure présente une densité inférieure à celle de la solution et en ce que l'analyte présente un seul élément complémentaire.

**7)** Procédé selon la revendication 1, caractérisé en ce que les particules de densité inférieure présentent un diamètre de 30 à 120 µm et les particules de densité supérieure présentent un diamètre inférieur à 10 µm.

**8)** Procédé selon la revendication 7, caractérisé en ce que les particules de densité inférieure présentent un diamètre de 40 à 100 µm et les particules de densité supérieure présentent un diamètre inférieur à 5 µm.

**9)** Procédé selon la revendication 1, caractérisé en ce que les particules de forte densité présentent un diamètre compris entre 10 et 50 µm et les particules de faible densité ont un diamètre inférieur 1 µm.

**10)** Procédé selon l'une des revendications 1 à 9, caractérisé en ce que les particules de faible et/ou de forte densité sont traitées pour être teintées ou fluorescentes.

**11)** Procédé selon l'une des revendications 1 à 10, caractérisé en ce que les éléments de reconnaissance sont choisis parmi :
- les sites antigéniques,
- les anticorps monoclonaux, les anticorps polyclonaux,
- les séquences spécifiques d'ADN ou d'ARN, naturels ou synthétiques,
- les sites spécifiques de récepteur ou de ligand.

**12)** Procédé selon l'une des revendications précédentes, caractérisé en ce que l'analyte est un antigène et les éléments de reconnaissance sont des anticorps monoclonaux ou polyclonaux.

**13)** Procédé selon la revendication 12, caractérisé en ce que les anticorps sont de type IgM, IgG, IgA, IgE.

**14)** Procédé selon la revendication 11, caractérisé en ce que l'analyte est une séquence d'ADN ou d'ARN monobrin et les éléments de reconnaissance sont des séquences complémentaires de ladite séquence.

**15)** Procédé selon l'une des revendications précédentes, caractérisé en ce que la phase solvant est choisie dans le groupe constitué par les tampons phosphate de sodium et potassium, bicarbonate de sodium, acétate de sodium, Tris-NaCl, MES ou mélanges alcool-tampon aqueux, mélanges diméthylsulfoxyde-tampon aqueux, diméthylformamide-tampon, dioxane-tampon aqueux ou solutions contenant des détergents (cationiques, anioniques ou zwitterions) destinés à mieux solubiliser l'analyte à partir de l'échantillon.

**16)** Procédé selon l'une des revendications précédentes, caractérisé en ce que les microbilles de densité supérieure sont constituées d'un matériau choisi dans le groupe constitué par les :
- particules fluorescentes
- particules magnétiques coloriées noir Fe₃O₄, rouge Fe₂O₃,
- pigments de cadmium rouge,
- pigments de cadmium jaune,
- pigments de phtalocyanine
- particules de latex-polystyrène bleues, rouges,
- pigments cosmétiques (à base de mica),
- pigments pour l'industrie automobile,
- or colloïdal
- Fractogel® TSK colorant triazine (bleu, rouge, vert, orange) de diamètre 32-63 µm.

**17)** Procédé selon l'une des revendications 1 à 16, caractérisé en ce que les microbilles de faible densité sont constituées d'un matériau choisi dans le groupe constitué par le :
- polyéthylène radicalaire,
- acrylamide,
- polystyrène expansé,
- matériau remplissage colonne chromatographie gaz-liquide, résines de polystyrène et de divinylbenzène, réticulées de diamètre 100-200 µm,
- billes de verre creuses.

**18)** Procédé selon l'une des revendications précédentes, caractérisé en ce que la masse surfacique d'éléments de reconnaissance et d'analyte est comprise entre 0,1 et 5 µg/cm².

**19)** Procédé selon l'une des revendications précédentes, caractérisé en ce que les microbilles teintées ont une densité comprise entre 200 et 600 % de la densité de la phase solvant et le diamètre compris entre 1 et 5 µm.

**20)** Procédé selon l'une des revendications précédentes, caractérisé en ce que les microbilles non teintées ont une densité comprise entre 10 et 30 % de la densité de la phase solvant et le diamètre compris entre 40 et 100 µm.

**21)** Dispositif pour mettre en oeuvre le procédé selon l'une des revendications 1 à 20, caractérisé en ce qu'il comporte un volume (4) tubulaire, présentant une zone inférieure et une zone supérieure (5) de lecture, limité à sa partie inférieure par une membrane (2) perméable aux liquides, dans lequel coulisse un piston (3), ledit volume contenant dans la zone inférieure une phase solide constituée des particules de densité supérieure et inférieure.

**22)** Dispositif selon la revendication 21, caractérisé en ce que la membrane est constituée d'un matériau choisi dans le groupe constitué par l'acétate de cellulose, le polyvinyldifluorobenzène, le nitrate de cellulose, la fibre de verre.

**23)** Dispositif selon la revendication 22, caractérisé en ce que le volume tubulaire est fixé verticalement et réversiblement à un récipient (7) contenant la solution dudit analyte.

**24)** Dispositif selon la revendication 23, caractérisé en ce que le volume traverse et est solidaire du bouchon (10) du récipient.

**25)** Dispositif pour mettre en oeuvre le procédé selon l'une des revendications 1 à 20, caractérisé en ce qu'il comporte un fourreau (12) dans lequel, dans sa partie supérieure, s'étendent un ou plusieurs tubes (26), chacun contenant une quantité appropriée de particules (35) et dans lequel dans sa partie inférieure, coulisse un réservoir (18) contenant la solution (44) à analyser de façon à ce que par translation, le réservoir permette à la solution de remplir les tubes et en ce que après contact entre les particules et la solution le résultat puisse être contrôlé dans la zone de lecture.

**26)** Dispositif selon la revendication 25, caractérisé en ce que le réservoir est limité par un film qui se déchire lorsqu'il rentre en contact avec les tubes sous l'effet du déplacement du piston.
